# EUROPEAN PATENT SPECIFICATION

(11) **EP 4 274 590 B1**
(45) Date of publication and mention of the grant of the patent: **18.11.2026**
(21) Application number: 21848258.6
(22) Date of filing: 29.12.2021
(51) Int. Cl.: A61K 35/744, A61K 35/747, A61P 15/02

(54) **LACTOBACILLUS CRISPATUS STRAIN LB714R FOR USE IN THE TREATMENT OF VAGINAL INFECTIONS**
LACTOBACILLUS CRISPATUS STAMM LB714R ZUR VERWENDUNG BEI DER BEHANDLUNG VAGINALER INFEKTIONEN
SOUCHE DE LACTOBACILLUS CRISPATUS LB714R POUR UTILISATION DANS LE TRAITEMENT DES INFECTIONS VAGINALES

(30) Priority: 05.01.2021 DK PA202100014; 29.04.2021 DK PA202100429
(43) Date of publication of application: 15.11.2023
(73) Proprietor: L'OREAL, 75008 Paris (FR)
(72) Inventor: VEDEL, Charlotte, 2100 Copenhagen Ø (DK); BILLEKOP OLSEN, Katja, 2100 Copenhagen Ø (DK); TESDORPF, Jens Edward, 2100 København Ø (DK); KJÆRULFF, Søren, 2100 København Ø (DK); ELVEBAKKEN, Helena Falholt, 2100 Copenhagen (DK)
(74) Representative: Larsen & Birkeholm A/S
(86) International application number: PCT/EP2021/087816
(87) International publication number: WO 2022/148699

(56) References cited:
- US-A1- 2013 171 253
- US-A1- 2016 184 372
- US-A1- 2016 279 182
- US-B2- 9 937 214

## Description

### Technical field of the invention

The present invention relates to a novel lactic acid bacterial strain, which alone or in combination can be used as probiotics. The invention also relates to pharmaceutical compositions and products for personal care comprising the strain alone or in combination. In particular, the present invention relates to use of the strain for prevention or treatment of vaginal infections or urogenital infections. The invention is defined by the appended claims.

### Background of the invention

Lower genital tract infections, including sexually transmitted infections (STIs) are some of the most common clinical problems among women of childbearing age. Vaginal discharge can be due to vaginal infections (yeast, bacterial vaginosis and trichomonas) or cervical infections (gonorrhoea or chlamydia). Additionally, there is a body of evidence linking vaginal infections to preterm delivery, low birth weight, and neonatal mortality, which are some of the most important problems faced in obstetrics.

Bacterial vaginosis (BV) is one of the most common genital infections in pregnancy often caused by the bacteria *Gardnerella vaginalis* (*G. vaginalis*)*.* Bacterial vaginosis is not a STI, but it can increase the risk of getting an STI. Women with bacterial vaginosis diagnosed during the second trimester of pregnancy are 40 percent more likely to give birth to a premature, low-birth weight infant than women without bacterial vaginosis. The prevention of even a small proportion of such births could translate into large monetary savings and a decrease in neonatal morbidity and mortality.

The vaginal microbiota, in particular lactic acid bacteria, play an important role in female health through modulation of immunity, countering pathogens and maintaining a pH below 4.5.

Furthermore, approximately, 90 % of the cases of the vulvovaginal candidiasis are due to *Candida spp.* predominated by *C*. *albicans* (Richter, S.S., et al. Antifungal Susceptibilities of Candida Species Causing Vulvovaginitis and Epidemiology of Recurrent Cases. J. Clin. Microbiol. 2005, Vol. 43, No. 5, pages 2155-2162). It is estimated that 75 % of women experience at least one episode in the course of their lifetime. Moreover, 25 % of the cases are recurrent with four or more episodes per year.

Infection often occurs after treatment with antibiotic therapy prescribed with a different therapeutic aim. It is also common in women taking oral contraceptives containing estrogens, in pregnant women and in women with diabetes. *Candida* infections are treated with antimycotics, such as triazole drugs (e.g. fluconazole, clotrimazole, myconazole, itraconazole) or nystatin. However, it is noteworthy that strains of *Candida glabrata* tend to display a high resistance against such treatments, while up to 20 % of the strains of *Candida albicans* isolated in clinics present resistance to fluconazole, one of the most typical treatments, stressing out the importance of finding new therapeutic tools for the management of vulvovaginal candidiasis.

An unhealthy vaginal microbiota or dysfunctional microbiota, e.g. caused by *Garnerella* species or *Candida* species, may be associated with infertility.

Lactic acid bacteria are a part of the microbial flora of the human gut, mouth, and vagina. Vaginal lactic acid bacteria play an important role in resistance to infection via production of acids and acidification of the vagina, by production of other antimicrobial products, such as hydrogen peroxide H₂O₂, antimicrobial peptides or biosurfactants.

It has been demonstrated that women with predominant vaginal lactic acid bacterial flora have a 50 percent lower frequency of gonorrhea and chlamydial infections, trichomoniasis and bacterial vaginosis. The presence of H₂O₂-producing lactic acid bacteria in the vagina have been linked to a decreased frequency of bacterial vaginosis, symptomatic yeast vaginitis and sexually transmitted pathogens including *Neisseria gonorrhea, Chlamydia trachomatis,* and *Trichomonas vaginalis.* In vitro studies have demonstrated that H₂O₂-producing lactic acid bacteria have potent bactericidal and viricidal properties against vaginal pathogens and even against human immunodeficiency virus (HIV).

Many women of childbearing age lack vaginal lactic acid bacteria. The vaginal ecosystem and microbiota are dynamically affected by medications, soaps, hygiene products, general health status, sexual practices and contraception. For some women, a healthy microbiota was never established at early life. Thus, a dysfunctional or unbalanced microbiota can also be established already at early life causing the problematic infections for the girls and women and also reducing fertility.

Many vaginal and systemic medications may kill vaginal lactic acid bacteria. Hence, treatment of infections with antibiotics may place women at increased risk for repeated acquisition of the infection or even development of antibiotic resistant infections.

Lactic acid bacteria for intravaginal or oral use have been available for over 50 years in the form of probiotic preparations available in health food stores, and acidophilus milk or yogurt in grocery stores. These products have included vaginal suppositories containing lyophilized *Lactobacillus spp.* These products have been largely non-efficacious in treating infections due to the failure of the products to colonize the vagina with the exogenous *Lactobacilli,* prevent biofilm formation of pathogens or to a lack of antimicrobial activity in vivo. Commercially available *Lactobacillus* has been shown to be unable to prevent biofilm formation of pathogens nor do the commercial *Lactobacillus* have antimicrobial activity against the persistent *G*. *vaginalis.*

Most commercially available probiotic strains are not systematically selected for their antimicrobial activity but chosen due to their ability to produce acids and to be upscaled (Kwak, Y-K. et al. 2017).

US 2016/184372 describes a composition comprising lactic bacteria for topical vaginal use and describes preventive and/or curative treatment of vaginal infections caused by vaginal pathogens, with particular reference to Gardnerella vaginalis and/or coliform bacteria, responsible for bacterial vaginosis. The *L. crispatus* ID 1605 is demonstrated to mediate an antagonistic action, which is less than what is demonstrated by *L. fermentum* (in particular *L. fermentum* LF15).

US 2016/279182 describes a method and a composition for treating bacterial vaginosis, where cervicovaginal secretions from a woman with vaginal microbiota dominated (>50%) by one of the species of lactobacillus typically found in the human vagina, like *Lactobacillus crispatus, L. iners, L. gasseri,* and/or *L jensenii,* is transplanted to women with bacterial vaginosis for restoring beneficial vaginal microbial communities and/or increasing resistance to sexually transmitted disease.

US 2013/171253 compositions for the oral and vaginal administration of human Lactobacilli (comprising the combination of *Lactobacillus crispatus, Lactobacillus rhamnosus, Lactobacillus jensenii* and *Lactobacillus gasseri*) for physiologic restoration of the vaginal flora, and treatment of asymptomatic bacterial vaginosis and prevention of preterm delivery caused through bacterial vaginosis.

US 9,937,214 describes a strain, *Lactobacillus crispatus* 262-1 which shows good acid and H₂O₂ production capability, good adhesion to the vaginal epithelial cells and significant resistance to bacterial vaginosis and various vaginal infections. The *Lactobacillus crispatus* 262-1 is described in the preparation of pharmaceuticals for preventing and/or treating gynaecological diseases and for the application in feminine care products such as medical instruments, disinfect products or cosmetics.

The ability of *G*. *vaginalis* to form biofilms contributes to the high rates of recurrence that are typical for BV and which unfortunately make repeated antibiotic therapy inevitable. Hence lactic acid bacteria which are able to prevent, inhibit or reduce the development of biofilm by *G*. *vaginalis* would be advantageous.

Further there is a need for lactic acid bacteria for the treatment of vaginal infections wherein the lactic acid bacteria maintain the antimicrobial activity, ability to acidify the vagina and prevent biofilm formation of *G*. *vaginosis* and/or *Candida spp.*

The present invention relates to a new isolated bacterium selected for its ability to specifically out compete the pathogenic microorganisms in the vagina and thereby reestablish a healthy microbiota. Further the new isolated bacteria is selected for its targeted antimicrobial activity towards *G*. *vaginalis* and/ or *Candida spp.*

### Summary of the invention

The invention is defined by the appended claims. An object of the present invention relates to a new lactic acid bacterium and its use in topical compositions, suited for the use in gynaecology and urology. Thus, an object of the present invention relates to a new isolated lactic acid bacterium which is able to either treat or prevent vaginal or urogenital infection, reduce recurrent infections, reduce STIs, improve fertility and/or reduce premature birth.

In particular, it is an object of the present invention to provide a new strain that solves the above-mentioned problems of the prior art with the ability to targeted control pathogens and thereby prevent infection.

The present invention relates to strains and compositions comprising the strain *Lactobacillus crispatus* LB714R deposited under accession number DSM 33732.

Thus, an aspect of the present invention relates to a composition comprising *Lactobacillus crispatus* LB714R, deposited under the accession number DSM 33732.

Another aspect of the present invention relates to a vaginal microbiome transplant comprising *Lactobacillus crispatus,* in particular comprising *Lactobacillus crispatus* LB714R, deposited under the accession number DSM 33732.

A method is described for preparing a vaginal microbiome transplant comprising the step of obtaining a microbiome sample from a female (such as a human female or an animal female), isolating a fraction from the microbiome sample comprising one or more *Lactobacillus* species, such as one or more *Lactobacillus crispatus* (in particular comprising *Lactobacillus crispatus* LB714R, deposited under the accession number DSM 33732), growing the fraction comprising one or more *Lactobacillus* species providing a growth fraction, and formulating the grown fraction into a suspension, spray, gel, cream, lotion, powder, capsule, ointment, oil, solution for lavages, ovules, a vaginal insert, a suppository, lozenge, tablets, microencapsulated product.

A method is described for preparing a vaginal microbiome transplant comprising the step of obtaining a microbiome sample from a female (such as a human female or an animal female) with healthy vaginal microbiota, isolate lactic acid bacteria from the microbiome sample (preferably the isolated lactic acid bacteria comprises one or more *Lactobacillus* species, such as one or more *Lactobacillus crispatus;* most preferably, the isolated lactic acid bacteria comprises one or more *Lactobacillus crispatus*)*,* selecting isolated bacteria which has antimicrobial activity against a pathogenic microorganism.

In an embodiment of the present invention the selected isolated bacteria which has antimicrobial activity against a pathogenic microorganism may be allowed to growth; and the selected isolated bacteria may be formulated into a suspension, spray, gel, cream, lotion, powder, capsule, ointment, oil, solution for lavages, ovules, a vaginal insert, a suppository, lozenge, tablets, microencapsulated product.

Yet an aspect of the present disclosure relates to a method for treating or preventing dysfunctional microbiota in a female, the method comprising the following steps:
1. Isolate lactic acid bacteria from the vagina of a female (such as a human female) with healthy vaginal microbiota;
2. Select isolated bacteria which has antimicrobial activity against a pathogenic microorganism;
3. Prepare a composition comprising one or more of the selected isolated bacteria; and
4. Transplant the composition to the vagina of female with a dysfunctional vaginal microbiota.

A further aspect of the present invention relates to an isolated bacterial strain selected from *Lactobacillus crispatus* LB714R, deposited under the accession number DSM 33732.

Yet an aspect of the present invention relates to a composition further comprising one or more bacterial strains selected from *Lactobacillus crispatus* LB714R, deposited under the accession number DSM 33732 for use as a medicament.

Still an aspect of the present invention relates to a composition comprising a bacterial strain selected from *Lactobacillus crispatus* LB714R, deposited under the accession number DSM 33732 for use in the prevention and/or treatment of a vaginal infection in a human or in an animal.

Another aspect of the present invention relates to a composition comprising a bacterial strain selected from *Lactobacillus crispatus* LB714R, deposited under the accession number DSM 33732for use in the improvement of fertility of a human or an animal.

Yet an aspect of the present invention relates to a composition comprising a bacterial strain selected from *Lactobacillus crispatus* LB714R, deposited under the accession number DSM 33732for use in reducing the risk of premature birth of a human or an animal.

A further aspect of the present invention relates to a composition comprising one or more vaginal isolate in particular, *Lactobacillus crispatus* LB714R, deposited under the accession number DSM 33732for use in the prevention, inhibition, or treatment of biofilm formation.

Still an aspect of the present invention relates to a medical device comprising the composition according to the present invention, or a vaginal microbiome transplant according to the present invention, or an isolated bacterial strain according to the present invention.

Yet an aspect of the present invention relates to a food ingredientand/or a personal hygiene product comprising the composition according to the present invention.

### Brief description of the figures

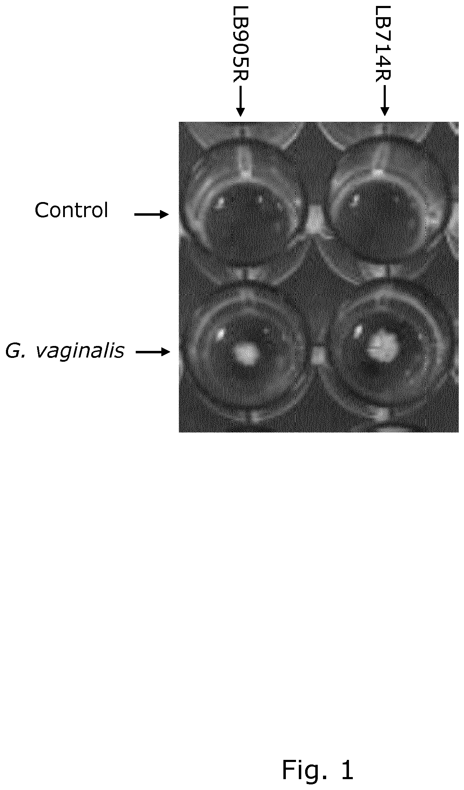

Figure 1 shows the co-aggregation of *G*. *vaginalis* with LB714R isolated from vagina of woman in late pregnancy, and

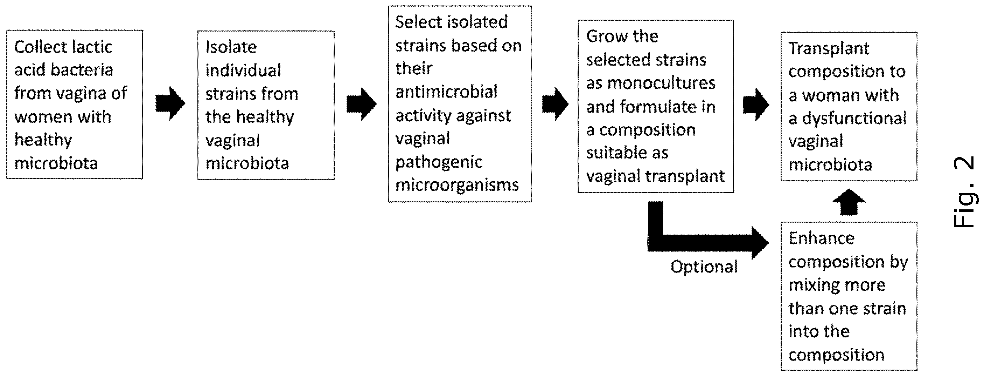

Figure 2 shows a preferred preparation of a composition or a vaginal microbiota transplant, including identification of suitable lactic acid bacteria capable of inhibiting *Gardnerella vaginalis,* such as one or more lactic acid bacteria, e.g. a *Lactobacillus* species, including one or more *Lactobacillus crispatus, Lactobacillus gasseri or Lactobacillus jensenii* (in particular, *Lactobacillus crispatus* LB714R, deposited under the accession number DSM *33732, Lactobacillus gasseri* LB905R deposited under accession number DSM 34094, *Lactobacillus crispatus* LB912R deposited under accession number DSM 34095, *Lactobacillus jensenii* LB918R deposited under accession number DSM 34096 or *Lactobacillus crispatus* LB919R deposited under accession number DSM 34097). The method includes transplantation of vaginal microbiota which involves a method for isolation and identification of strains for prevention or treatment of vaginal infections or urogenital infections. In particular the method includes (i) collection of lactic acid bacteria from women having a healthy vaginal microbiota; (ii) isolate individual strains from the healthy vaginal microbiota; (iii) select isolated strains based on their antimicrobial activity against vaginal pathogenic microorganisms; (iv) grow the selected stains as monocultures and formulate in a composition suitable for vaginal transplant; (v) optionally enhance composition by mixing more than one strain into the composition; and (vi) transplant composition to a woman with a dysfunctional vaginal microbiota,

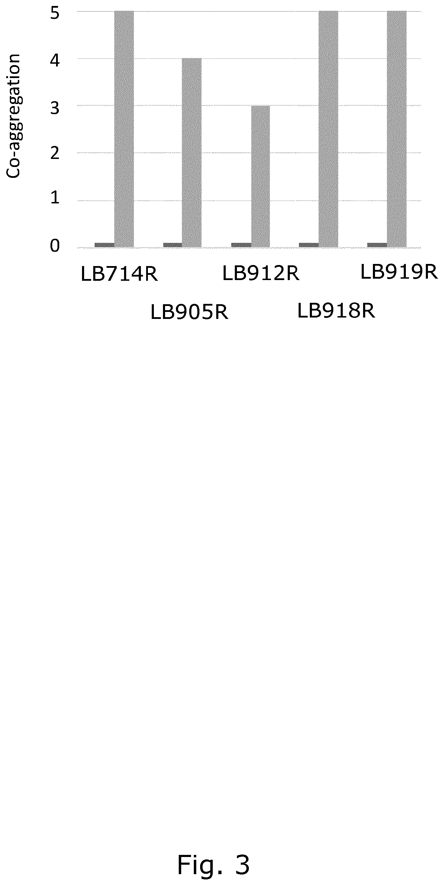

Figure 3 shows co-aggregation scores of *Lactobacillus crispatus* LB714R, deposited under the accession number DSM 33732, *Lactobacillus gasseri* LB905R deposited under accession number DSM 34094, *Lactobacillus crispatus* LB912R deposited under accession number DSM 34095, *Lactobacillus jensenii* LB918R deposited under accession number DSM 34096 and *Lactobacillus crispatus* LB919R deposited under accession number DSM 34097 according to the present invention illustrated by the light gray columns determined according to Cisar, J. O. et al. (1979).

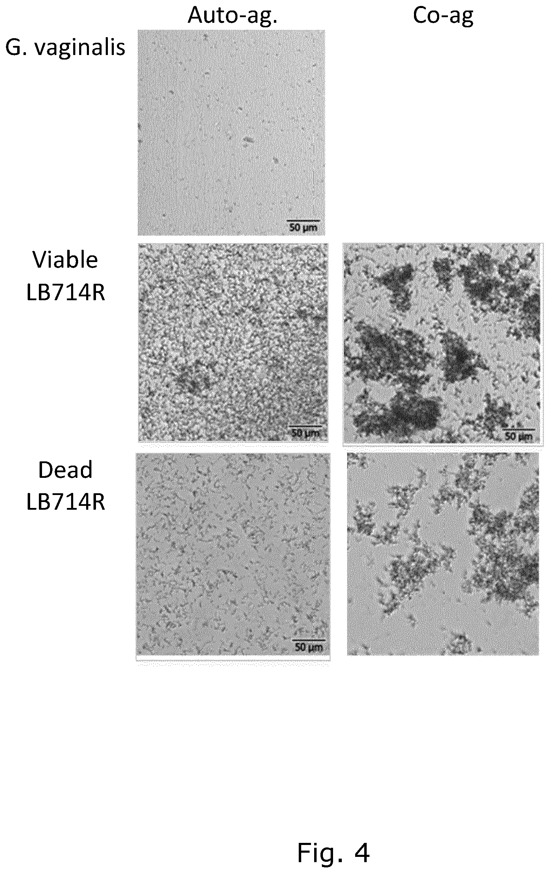

Figure 4 shows microscope images of the ability of *Lactobacillus crispatus* LB714R, deposited under the accession number DSM 33732, to co-aggregate with G. vaginalis using viable and dead/inactivated cells of the *Lactobacillus* strain, *Lactobacillus crispatus* LB714R.

The present invention will now be described in more detail in the following.

### Detailed description of the invention

Accordingly, the inventors of the present invention found specific lactic acid bacteria having antimicrobial activity towards normalizing an unhealthy or a dysfunctional vaginal microbiota in a human or an animal, and/or inhibition of biofilm formation, e.g. on a surface as defined herein.

In an embodiment of the present invention the animal may include, but are not limited to, primates, farm animals, sport animals, rodents and pets. More specifically the animals may include mice, rats, hamsters, and guinea pigs; rabbits; dogs; cats; sheep; pigs; piglets; sows; poultry; turkeys; broilers; minks; goats; cattle; horses; and non-human primates such as apes and monkeys.

The lactic acid bacteria may preferably be a probiotic bacteria, and preferably a probiotic bacteria not previously identified.

The new probiotic bacteria may reduce number of infections in the lower genital tract and thereby improve fertility, reduce risk of premature birth and reduce risk of STIs.

The lower genital tract may preferably include the urinary tract and/or the vagina. The vagina may be the elastic, muscular part of the female genital tract. In humans, it extends from the vulva to the cervix.

The one or more lactic acid bacteria according to the present invention may have antimicrobial activity.

The lactic acid bacteria according to the present invention may be able to colonize mucous membranes and/or prevent biofilm formation by pathogenic vaginal microorganisms and/or have antimicrobial activity against pathogenic vaginal microorganisms.

A "mucous membrane" or "mucosa" is a membrane that lines various cavities in the body and covers the surface of internal organs. It consists of one or more layers of epithelial cells overlying a layer of loose connective tissue. It is mostly of endodermal origin and is continuous with the skin at various body openings such as the eyes, ears, inside of the nose and mouth, lip, vagina the urethral opening and the anus.

The composition according to the present invention may be used for treating a mucous membrane condition in a human or an animal in need thereof, comprising applying to a mucous membrane of the composition comprising at least one lactic acid bacteria according to the present invention.

In a preferred embodiment of the present invention the lactic acid bacteria according to the present invention may be selected from *L. crispatus* LB714R (DSM 33732).

Preferably, the lactic acid bacteria according to the present invention may comprise at least one or more *Lactobacillus crispatus,* in particular *Lactobacillus crispatus* LB714R (DSM 33732).

*Lactiplantibacillus plantarum,* was previously named *Lactobacillus plantarum,* and is a member of the genus *Lactiplantibacillus* and commonly found in many fermented food products as well as anaerobic plant matter.

The present invention relates to the use of one or more lactic acid bacteria according to the present invention for preparing a pharmaceutical composition for preventing and/or treating vaginal infections.

Furthermore, the present invention relates to the use of one or more lactic acid bacteria according to the present invention for preparing a pharmaceutical composition for preventing and/or treating urinary-tract infections.

The present invention also relates to the use of one or more lactic acid bacteria according to the present invention for preparing a pharmaceutical composition for preventing and/or treating STIs.

Moreover, the present disclosure relates to the use of one or more lactic acid bacteria according to the present disclosure for preparing a pharmaceutical composition for restoring a dysfunctional vaginal microbiota.

A "dysfunctional microbiota" or "dysfunctional vaginal microbiota" can be determined by either the Nugent score or a diagnosis done by evaluating the pH, the presences of *Lactobacillus spp.* versus a mixed flora consisting of *Gardnerella vaginalis,* Bacteroides species or *Candida* species or by use of the Amsel Criteria for bacterial vaginosis which includes pH, evaluating the presence of clue cells, white discharge and an odour of amines after mixing with KOH.

The one or more lactic acid bacteria according to the invention may be used for preparing a composition for restoring a dysfunctional vaginal microbiota to be combined with an antibiotic treatment or to restore unbalanced microbiota after antibiotic treatment.

An embodiment of the present disclosure relates to the use of one or more lactic acid bacteria according to the disclosure for preparing a composition for improving fertility and/or for preparing a composition for reducing risk of premature birth.

The composition according to the present invention may be formulated into medical device. Thus, the composition according to the present invention comprising one or more lactic acid bacteria according to the invention, may be used as a medical device.

The composition according to the present invention may be used for the treatment or prevention of microbial imbalances in mucous membranes in a human or an animal comprising administration, to the human or animal, an effective amount of lactic acid bacteria according to the invention.

A method may be described for isolating lactic acid bacteria from the vaginal microbiota of healthy women comprising the following steps:
1. Isolate lactic acid bacteria from women with healthy vaginal microbiota
2. Select isolated bacteria with has antimicrobial activity against either *Garnerella vaginalis* and/ or *Candida albicans*
3. Prepare a composition comprising at least one of the selected isolated bacteria and
4. Use the prepared composition according to the invention

The term "lactic acid bacteria" includes species from the families Lactobacillaceae, Aerococcaceae, Bifidobacteriaceae, Carnobacteriaceae, Enterococcaceae, Leuconostocaceae and Streptococcaceae. These lactic acid bacteria are considered non-pathogenic and are used as probiotic bacteria in general to improve gastrointestinal flora and in the treatment of gastrointestinal symptoms. "Probiotics" may be defined as live microorganisms that, when administrated or consumed in adequate quantities, confer health benefits on the host.

The lactic acid bacteria according to the present invention may be provided in viable or non-viable form.

The non-viable cells according to the present invention may be provided as a lysate, as a fraction, as a ferment, as metabolites, as an extract, as a derivative, as analogues, as postbiotics, as paraprobiotics or as a mutant of the *Lactobacillus crispatus* strains according to the present invention.

"Lysates", "derivatives", "analogues", "fractions", "postbiotics", "paraprobiotics" or "extracts" may be obtained from dead or killed lactic acid bacteria. These lysates, fractions, derivative, analogues, and extracts preferably have the properties of being able to bind or co-aggregate with pathogenic bacteria thereby preventing growth and/or biofilm formation of a pathogen, where "lysate" as well as the term "extract" refers in particular to a solution or suspension in an aqueous medium of the cells of the microorganism and/or metabolites according to the invention and comprises, for example, macromolecules such as DNA, RNA, proteins, peptides, lipids, carbohydrates, cell wall material etc. as well as cell detritus. The lysate preferably includes the cell wall or cell wall constituents including binding receptors for co-aggregation. Methods of producing lysates are sufficiently well known to those skilled in the art and includes, for example, the use of a French press or enzymatic lysis, a ball mill with glass beads or iron beads. Cells can be broken open by enzymatic, physical or chemical methods. Examples of enzymatic cell lysis may include individual enzymes as well as enzyme cocktails, for example, proteases, proteinase K, lipases, glycosidases; chemical lysis may be induced by ionophores, detergents such as SDS, acids or bases; physical methods may also be implemented by using high pressures such as the French press, osmolarities, temperatures or alternating between heat and cold. Furthermore chemical, physical and enzymatic methods may of course be combined. "Extract" can be any of these cellular components, metabolites, ferment or cell fractions.

In another aspect, the present teachings disclose a composition substantially free from viable microorganisms. The composition can comprise cell material including dead cells in form of a lysate or ferment. In one aspect of the invention the composition comprises the supernatant from fermentation and cell material having a further functional effect. In one aspect of the invention the cell material is capable of co-aggregating with the target pathogen or inhibit the target pathogen.

The microorganisms according to the invention are preferably in isolated or purified form, where the term "isolated" means in particular that the lactic acid bacteria are derived from their culture medium including their natural medium.

An embodiment of the present invention relates to a composition comprising live lactic acid bacteria according to the present invention for use in treating; alleviating, suppressing; prophylaxis; and/or preventing growth of a pathogenic microorganism.

Preferably, the present invention may provide a composition as defined herein for use in the treatment, alleviating, suppressing; prophylaxis and/or prevention of one or more pathogenic microbial infection in a human or an animal.

Preferably, the present invention may provide a composition as defined herein for use in the treatment, alleviating, suppressing; prophylaxis and/or prevention of one or more fungal or bacterial infection in a human or an animal. More preferably the present invention may provide a composition as defined herein for use in the treatment, alleviating, suppressing; prophylaxis and/or prevention of growth of a pathogenic microorganism selected from *Gardnerella vaginalis, Candida albicans* or both.

In an embodiment of the present invention the treatment, alleviating, suppressing; prophylaxis and/or prevention of growth may be determined as a decrease in growth of at least 25 percent. Preferably the decrease in growth may be at least 50 percent, more preferably the decrease in growth may be at least 80%, more preferably the decrease in growth may be at least 90%.

Thus, an aspect of the present invention relates to a composition comprising *Lactobacillus crispatus* LB714R, deposited under the accession number DSM 33732.

In an embodiment of the present invention the composition may further comprise a lactic acid bacterial strain selected from one or more *Pediococcus* strains (such as *Pediococcus pentosaceus LB606R,* deposited under the deposit accession number DSM 33730); and/or one or more *Lactobacillus* strains (preferably a *Lactobacillus plantarum,* such as *Lactobacillus plantarum* LB356R (deposited under the accession number DSM 33094) Preferably, the composition according to the present invention may further comprise *Pediococcus pentosaceus* LB606R, deposited under the accession number DSM 33730.

Thus, in an embodiment of the present disclosure, the composition comprises the combination of:
- one or more *Lactobacullus crispatus* (preferably, *Lactobacillus crispatus* LB714R, deposited under the accession number DSM 33732);
- one or more *Pediococcus* strains (preferably, *Pediococcus pentosaceus LB606R,* deposited under the deposit accession number DSM 33730); and
- one or more *Lactobacillus plantarum* (preferably *Lactobacillus plantarum* LB356R, deposited under the accession number DSM 33094)).

In an embodiment of the present invention the composition prevents or inhibits biofilm formation.

In yet an embodiment of the present invention, the composition prevents or inhibits biofilm formation. In particular, the composition of the present invention prevents or inhibits biofilm formation of one or more bacteria. More specifically, the composition of the present invention prevents or inhibits biofilm formation of pathogenic *Garnerella* species. Even more specifically, the composition of the present invention prevents or inhibits biofilm formation of *Garnerella vaginalis.*

In the present context the inhibition of biofilm formation may be an at least 25% biofilm inhibition, such as an at least 50% biofilm inhibition, e.g. an at least 75% biofilm inhibition, such as an at least 85% biofilm inhibition, e.g. an at least 90% biofilm inhibition, such as an at least 95% biofilm inhibition, e.g. an at least 98% biofilm inhibition.

The term "prevent" is art-recognized, and when used in relation to a condition, such as a local recurrence, is well understood in the art, and includes administration of a composition which reduces the frequency of, or delays the onset of, symptoms of a medical condition in a subject relative to a subject which does not receive the composition. Thus, prevention of infection includes, for example, reducing the number of detectable pathogenic microorganisms in a population of patients receiving a prophylactic treatment relative to an untreated control population, and/or delaying the appearance of detectable lesions in a treated population versus an untreated control population, e.g., by a statistically and/or clinically significant amount.

The term "inhibition" or "inhibit" as used herein, means the killing of a microorganism, such as an undesired microorganism, or the control of the growth of said microorganism. Including inhibition of biofilm formation which can be inhibition of the initial attachment of a microorganism or inhibition of the further formation of a biofilm by growth inhibition of the biofilm forming microorganism.

The inventors of the present invention identified and isolated the specific strains of *Lactobacillus crispatus* (LB714R/DSM 33732) and identified one or more specific activity provided by the specific strain, like inhibition or prevention of biofilm formation, and/or for the treatment of dysfunctional vaginal microbiota.

As used herein, the term "treating" or "treatment" includes reversing, reducing, or arresting the symptoms, clinical signs, and underlying pathology of a condition in a manner to improve or stabilize a subject's condition.

As used herein, and as well-understood in the art, "treatment" is an approach for obtaining beneficial or desired results, including clinical results. For purposes of this subject matter, beneficial or desired clinical results include, but are not limited to, alleviation or amelioration of one or more symptoms, diminishment of extent of disease, stabilized (i.e., not worsening) state of disease, prevention of disease, delay or slowing of disease progression, and/or amelioration or palliation of the disease state.

The decrease can be a 10% decrease in severity of complications or symptoms, 20%, 30%, 40%, 50%, 60%, 70%, 80%, 90%, 95%, 98%, 99% or 100% decrease in severity of complications or symptoms.

In an embodiment of the present invention, the composition according to the present invention may be used for inhibiting or preventing biofilm formation on a surface. Preferably, the surface may be a surface in a hospital, and/or a surface of a medical device.

In yet an embodiment of the present invention, the composition according to the present invention may be used for inhibiting biofilm formation in or on the human or animal body.

In the present context, biofilm formation may be produced by one or more microorganism. In particular, the one or more microorganisms may be a bacteria. The one or more bacteria may be one or more pathogenic *Gardnerella* species. The one or more pathogenic *Gardnerella* species may be one or more *Gardnerella vaginalis.*

In an embodiment of the present invention the composition may be formulated for oral use, topical use, anal use or genital use.

Preferably, the composition according to the present invention is formulated for oral or genital use. Even more preferably, the composition according to the present invention is formulated for genital use.

In an embodiment of the present invention the genital use may be male genital use, or female genital use, such as vaginal and/or urinary use.

During topical use of the composition of the present invention the composition may be used on the skin, on the mucosa, or on the nails of a human or animal.

The composition may be formulated into a suspension, spray, gel, cream, lotion, powder, capsule, ointment, oil, solution for lavages, ovules, a vaginal insert, a suppository, lozenge, tablets, microencapsulated product. The skilled person would know how to prepare such formulation.

In an embodiment of the present invention the *Lactobacillus crispatus* LB714R, deposited under the accession number DSM 33732) may be provided as viable cells, as non-viable cells, as a lysate, as a fraction, as a ferment, as metabolites, as an extract, as a derivative, as analogues, or as a mutant .

Preferably, the *Lactobacillus crispatus* LB714R, deposited under the accession number DSM 33732 may be provided as viable cells, as non-viable cells, cell fractions or as a lysate.

A mutant of one of the *Lactobacillus* strains according to the present invention may be provided by a method to obtain a mutant of the *Lactobacillus crispatus* LB714R, deposited with Germena Collection of Microorganisms and Cell Cultures under the accession number DSM 33732, comprising using the deposited strain as starting material and applying mutagenesis, wherein the obtained mutant retains or enhances the probiotic and/or antibacterial and/or antimycotic and/or antimicrobial properties and/or the capacity to inhibit, prevent or treat bacterial and/or fungal vaginosis.

In an embodiment of the present invention the composition further comprises one or more prebiotic.

"Prebiotics" are non-digestible food components that increase the growth of specific microorganisms. "Synbiotics" are compositions comprising at least one probiotic and at least one prebiotic. Such compositions are understood to encourage the growth of beneficial bacteria (e.g. the probiotic). Thus, powerful synbiotics are based on a combination of specific strains of probiotic bacteria with carefully selected prebiotics. They can lead to an important health benefit to a human or an animal.

Prebiotics refer to chemical products that induce the growth and/or activity of commensal microorganisms (e.g., bacteria and fungi) that contribute to the well-being of their host. Prebiotics are nondigestible carbohydrates that are undigested or partly un-digested by the host and stimulate the growth and/or activity of advantageous bacteria that colonize the host.

Some oligosaccharides that are used as prebiotics are fructo-oligosaccharides (FOS), xylooligosaccharides (XOS), polydextrose, pectins, galacto-oligo saccharides (GOS) or human milk oligosaccharides (HMO). Moreover disaccharides like lactulose or lactose some monosaccharides such as tagatose can also be used as prebiotics.

In an embodiment of the present invention a composition may be provided comprising a probiotic microorganism (e.g. a lactic acid bacteria according to the present invention) and at least one more active ingredient, and optionally in combination with a prebiotic microorganism.

The other active ingredient (or other ingredients) is not limited in any way.

In a preferred aspect, at least one prebiotic compound is comprised in the composition of the invention, i.e. as other ingredient. In a very broad concept, prebiotics are all those compounds which can be metabolized by probiotics. Preferably prebiotics are non-digestible or poorly digestible by a human or an animal. Prebiotics are well known in the art and when used in the present invention there is no particular limitation of the prebiotic as such. In preferred embodiments however the at least one prebiotic product in the composition is selected from the following compounds and compositions: non-digestible carbohydrates, beta-glucans, mannan-oligosaccharides, inulin, oligofructose, human milk oligosaccharides (HMO), galactooligosaccharides (GOS), lactulose, lactosucrose, galactotriose, fructo-oligosaccaride (FOS), cellobiose, cellodextrins, cylodextrins, maltitol, lactitol, glycosilsucrose. Optionally, mannan-oligosaccharides and/or inulin may be preferred.

HMOs include lacto-N-tetraose, lacto-N-fucopentaose, lacto-N-triose, 3'-sialyllactose, lacto-N-neofucopentaose, sialic acid, L-fucose, 2-fucosyllactose, 6'-sialyllactose, lacto-N-neotetraose and 3-fucosyllactose.

D- and L-fucose strengthen natural defense of skin, stimulate epidermis immune defense and/or prevent and/or treat cutaneous autoimmune disease. In one preferred embodiment of the invention the composition comprises D- or L-fucose.

In an embodiment of the invention the composition further comprises L-fucose in a concentration in the range of 1 mM to 1000 mM, such as in the range of 10 mM to 500 mM, e.g. in the range of 25 mM to 250 mM.

Preferably, the composition according to the present invention comprising 10³ to 10¹³ colony forming units of lactic acid bacteria according to the present invention per gram. More specifically a pharmaceutical composition comprising 10⁶ to 10¹² colony forming units of lactic acid bacteria per gram. More specifically a pharmaceutical composition comprising 10⁷ to 10¹¹ colony forming units of lactic acid bacteria per gram.

The number of microorganisms may be measured as Colony Forming Units CFU/ml.

In an embodiment of the present invention the composition may be a pharmaceutical composition. Preferably a pharmaceutical composition comprising one or more lactic acid bacteria according to the present invention together with a pharmaceutically acceptable carrier and/or diluent.

The pharmaceutical composition may be in the form of a suspension, spray, gel, cream, lotion, powder, capsule, oil, solution for lavages, ovules, a vaginal insert, a suppository, lozenge, tablets, or a microencapsulated product.

The composition may be provided with a therapeutic effective amount of the *Lactobacillus crispatus* LB714R, deposited under the accession number DSM 33732).

In the context of the present invention the terms "therapeutic effective amount" or "effective amount" may refer to an amount of the compounds in a composition or preparation which, when administered as part of a desired dosage regimen (to a human or an animal, preferably a human) alleviates a symptom, ameliorates a condition, or slows the onset of disease conditions according to clinically acceptable standards for the disorder or condition to be treated or the cosmetic purpose, e.g., at a reasonable benefit risk ratio applicable to any medical treatment.

For the practical use, the microorganisms of the invention may be formulated in suitable administration forms such as gels, creams, capsules, tablets, solutions for lavages, sachets, any food products and the like. Unit doses may comprise from 10 to 10¹³ cells of each single strain, the preferred dosage being above 10⁵ cells per unit dose. More preferable dosage is above 10⁶ cells per unit dose.

The bacterial cultures may be stabilized in freeze dried, lyophilized or microencapsulated forms and may be prepared according to conventional methods.

For the preparation of aqueous formulations for lavages and irrigations a two-phase system can be used. E.g. small bottles with reservoirs containing the lyophilized microorganisms, to be dissolved before use in a suitable liquid carrier contained in the bottles.

For the preparation of creams and gels, the lactic acid bacteria are stabilized in the formulation. Preferable in formulations with low water activity or in a microencapsulated form.

In yet an embodiment of the present invention the composition may be a vaginal microbiome transplant.

A vaginal microbiome transplant may be a transplantation of microbiota from a healthy female to an unhealthy vaginal microbiota or a dysfunctional vaginal microbiota. Thus, in an embodiment of the present invention the vaginal microbiome transplant may be obtained from a healthy female, in particular, from the vagina of a healthy female; and transferred to another female, in particular transferred to the vagina of another female, preferably another female having an unhealthy vaginal microbiota or a dysfunctional vaginal microbiota.

In an embodiment of the present invention the vaginal microbiome transplant may comprise at least *Lactobacillus crispatus* LB714R, deposited under the accession number DSM 33732.

The vaginal microbiome transplant may further comprise a lactic acid bacterial strain selected from one or more *Pediococcus* strains (such as *Pediococcus pentosaceus* LB606R, deposited under the deposit accession number DSM 33730).

The vaginal microbiome transplant may further comprise a lactic acid bacterial strain selected from one or more *Lactobacillus plantarum,* (such as *Lactiplantibacillus plantarum* LB679R, deposited under the deposit accession number DSM 33731; *Lactobacillus plantarum* LB356R, deposited under the accession number DSM 33094; *Lactobacillus plantarum* LB244R, deposited under the accession number DSM 32996; and any combination hereof).

Preferably, the vaginal microbiome transplant may be formulated as a suspension, spray, gel, cream, lotion, powder, capsule, oil, solution for lavages, ovules, a vaginal insert, a suppository, lozenge, tablets, microencapsulated product.

In an embodiment of the present invention the composition and/or the vaginal microbiome transplant according to the present invention does not comprise an antibiotic compound.

Antibiotic compounds may preferably not be included into the composition and/or the vaginal microbiome transplant according to the present invention because such compound may unspecifically kill both healthy and pathogenic bacteria and thus also destroys the desired and healthy microbiota.

Antibiotic compounds are a type of antimicrobial substances being active against bacterial growth either by inhibiting growth or by killing the bacteria.

The vaginal microbiome transplant according to the present invention may be prepared by a method for preparing a vaginal microbiome transplant comprising the step of obtaining a microbiome sample from a female (such as a human female or an animal female), isolating a fraction from the microbiome sample comprising *Lactobacillus crispatus* LB714R, deposited under the accession number DSM 33732), growing the fraction comprising one or more *Lactobacillus* species providing a growth fraction, and formulating the grown fraction into a suspension, spray, gel, cream, lotion, powder, capsule, ointment, oil, solution for lavages, ovules, a vaginal insert, a suppository, lozenge, tablets, microencapsulated product.

In an embodiment of the present invention the female from which the microbiome sample may be obtained is preferably a pregnant female. Preferably, the pregnant female is 7 months or less from given birth when the microbiome sample may be obtained, such as 5 months or less, e.g. 3 months or less, such as 1 months or less, e.g. 20 days or less, such as 10 days or less, e.g. 5 days or less.

The present invention relates to a method for treating or preventing dysfunctional microbiota in a female, the method comprising the following steps:
1. Isolate lactic acid bacteria from the vagina of a female (such as a human female) with healthy vaginal microbiota;
2. Select isolated bacteria which has antimicrobial activity against a pathogenic microorganism;
3. Prepare a composition comprising one or more of the selected isolated bacteria; and
4. Transplant the composition to the vagina of female with a dysfunctional vaginal microbiota.

Furthermore, the present invention relates to a composition comprising (or consisting essentially of) a combination of a first lactic acid bacterial strain and a second lactic acid bacterial strain, wherein the first lactic acid bacterial strain may be characterized by inhibition of *Gardnerella vaginalis* and the second lactic acid bacterial strain may be characterized by inhibition of *Candida albicans.*

In an embodiment of the present invention the composition comprising a combination of a first strain and a second strain may be a vaginal microbiome transplant used in a vaginal microbiome transplant.

The present invention also relates to a method for selecting one or more lactic acid bacterial strains for vaginal transplantation comprising the step of selecting at least one first lactic acid bacterial strain characterized by inhibition of *Gardnerella vaginalis* and selecting at least one second strain characterized by inhibition of *Candida albicans.*

The first lactic acid bacterial strain and the second lactic acid bacterial strain may be the same or may be different lactic acid bacterial strains. Preferably, the first lactic acid bacterial strain and the second lactic acid bacterial strain may be different lactic acid bacterial strains.

The first strain comprises (or consisting essentially of) *Lactobacillus crispatus* LB714R, deposited under the accession number DSM 33732).

The second strain may comprise (or consisting essentially of) one or more lactic acid bacteria. Preferably, the one or more lactic acid bacteria of the second strain may be selected from one or more *Pediococcus* strains (preferably, *Pediococcus pentosaceus* LB606R, deposited under the deposit accession number DSM 33730); one or more *Lactobacillus* strains (preferably a *Lactobacillus plantarum,* such as *Lactobacillus plantarum* LB356R (deposited under the accession number DSM 33094); or any combination hereof.

The second strain may preferably comprise (or consisting essentially of) one or more lactic acid bacteria selected from one or more *Pediococcus* strains, in particular *Pediococcus pentosaceus* LB606R, deposited under the deposit accession number DSM 33730. Preferably, inhibition of *Gardnerella vaginalis* and/or *Candida albicans* may be determined by the inhibition reduction in biofilm formation as described herein.

The present disclosure also relates to a method for providing a composition comprising a combination of a first lactic acid bacterial strain and a second lactic acid bacterial strain, comprises the steps of:
(a) selecting at least one first lactic acid bacterial strain characterized by inhibition of *Gardnerella vaginalis;*
(b) selecting at least one second lactic acid bacterial strain characterized by inhibition of *Candida albicans;* and
(c) mixing the selected at least one first lactic acid bacterial strain obtained in step (a) with the selected at least one second lactic acid bacterial strain obtained in step (b), providing the composition comprising a combination of a first lactic acid bacterial strain and a second lactic acid bacterial strain.

In an embodiment of the present invention the pathogenic microorganism against which the isolated bacteria show antimicrobial activity step 2 of the above method, may be selected from *Gardnerella vaginalis, Candida albicans* or both.

A preferred embodiment of the present invention relates to an isolated *Lactobacillus crispatus* LB714R, deposited under the accession number DSM 33732.

The microorganisms according to the present invention may preferably be in isolated or purified form, where the term "isolated" means in particular that the lactic acid bacteria are derived from their culture medium including their natural medium, for example. The term "purified" is not restricted to absolute purity.

A preferred embodiment of the present invention relates to an isolated bacterial strain selected from *Lactobacillus crispatus* LB714R, deposited under the accession number DSM 33732, wherein said strains presents antimicrobial activity.

The antimicrobial activity may be normalizing an unhealthy or a dysfunctional vaginal microbiota in a human or an animal, and/or inhibition of biofilm formation on a surface as defined herein. In particular, the isolated bacterial strain selected from *Lactobacillus crispatus* LB714R, deposited under the accession number DSM 33732,may inhibit biofilm formation of one or more bacteria. More specifically, the isolated strain may inhibit biofilm formation of pathogenic *Gardnerella* species. Even more specifically, the isolated strain may inhibit biofilm formation of *Gardnerella vaginalis.*

The antimicrobial activity may include treatment of sexually transmitted infections (STI); vaginal infections (like bacterial vaginosis) or cervical infections (like gonorrhoea or chlamydia). Even bacterial vaginosis may not directly be considered a sexually transmitted infection, it may increase the incidence of sexually transmitted infections and treatment of bacterial vaginosis may be considered at least an indirect treatment of sexually transmitted infections.

A preferred embodiment of the present invention relates to a composition comprising one or more bacterial strains selected from the species *Lactobacillus crispatus* LB714R, deposited under the accession number DSM 33732 for use as a medicament.

In yet an embodiment of the present invention the composition consisting essentially of one or more bacterial strains selected *Lactobacillus crispatus* for use as a medicament.

In the context of the present invention, the term "consisting essentially of", relates to a limitation of the scope of a claim to the specified features or steps and those features or steps, not mentioned and that do not materially affect the basic and novel characteristic(s) of the claimed invention.

A preferred embodiment of the present invention relates to a composition comprising a bacterial strain selected from *Lactobacillus crispatus* LB714R, deposited under the accession number DSM 33732for use in the prevention and/or treatment of a vaginal infection in a human or in an animal.

The composition according to the present invention may be further supplemented one or more *Pediococcus* strains; and/or one or more bacteria strains selected from one or more *Lactiplantibacillus* strains. This, combination of one or more *Lactobacillus crispatus,* and one or more *Pediococcus* strains, and/or one or more *Lactiplantibacillus* strains, may further improve the effect of the composition in preventing and/or treatment of a vaginal infection, in a human or in an animal.

The composition according to the present invention may decrease the pH-value of the vagina. In an embodiment of the present invention the pH of the vagina is decreased to a pH-value of pH 6 or below, such as a pH-value of pH 5 or below, e.g. a pH-value of pH 4.5 or below, such as a pH-value of pH 4 or below, e.g. a pH-value in the range of pH 3-5, such as in the range of pH 3.5-4.5.

The vaginal infections may include or cause bacterial vaginosis, microbial imbalance of the vagina, urinary tract infections, reduced fertility, and/or increased risk of premature births.

An embodiment of the present invention relates to a composition for use in the prevention and/or treatment of bacterial vaginosis.

A further embodiment of the present invention relates to a composition for use in the prevention and/or treatment of microbial imbalance of the vagina.

Yet An embodiment of the present invention relates to a composition for use in the prevention and/or treatment of urinary-tract infections.

A preferred embodiment of the present invention relates to a composition comprising *Lactobacillus crispatus* LB714R, deposited under the accession number DSM 33732 for use in the improvement of fertility of a human or an animal.

A further preferred embodiment of the present invention relates to a composition comprising *Lactobacillus crispatus* LB714R, deposited under the accession number DSM 33732 for use in reducing the risk of premature birth of a human or an animal.

In an embodiment of the present invention a composition comprising *Lactobacillus crispatus* LB714R, deposited under the accession number DSM 33732, may be used in the improvement of fertility of a human or an animal and/or in reducing the risk of premature birth of a human or an animal.

Preferably, the prevention and/or treatment of bacterial vaginosis in a human or in an animal may be vaginal prevention and/or treatment of bacterial vaginosis; oral prevention and/or treatment of bacterial vaginosis; and/or intestinal prevention and/or treatment of bacterial vaginosis.

The present invention may relate to a composition consisting essentially of *Lactobacillus crispatus* LB714R, deposited under the accession number DSM 33732), for use in the prevention and/or treatment of vaginal infections in a human or in an animal.

A preferred embodiment of the present invention relates to a composition comprising *Lactobacillus crispatus* LB714R, deposited under the accession number DSM 33732) for use in the prevention, inhibition, or treatment of biofilm formation.

The biofilm formation may be a bacterial biofilm formation or a yeast or fungal biofilm formation, such as a pathogenic *Garnerella* species biofilm formation; in particular a *Gardnerella vaginalis* biofilm formation.

In an embodiment of the present invention the composition according to the present invention may comprise *Lactobacillus crispatus* LB714R, deposited under the accession number DSM 33732) according to the present invention, in combination with one or more *Pediococcus* strains (in particular, *Pediococcus pentosaceus* LB606R, deposited under the deposit accession number DSM 33730), or one or more *Lactobacillus plantarum* (in particular, *Lactobacillus plantarum* LB356R, deposited under the accessionaccession number DSM 33094; and/or *Lactobacillus plantarum* LB244R, deposited under the accessionaccession number DSM 32996) for use in the prevention, inhibition, or treatment of bacterial biofilm formation, fungal biofilm formation and/or yeast biofilm formation.

Such yeast biofilm formation may be a yeast biofilm formation provided by one or more pathogenic *Candida* species; in particular *Candida albicans.*

The composition according to the present invention may consisting essentially of *Lactobacillus crispatus* LB714R, deposited under the accession number DSM 33732) according to the present invention, optionally, in combination with one or more *Pediococcus* strains (in particular, *Pediococcus pentosaceus* LB606R, deposited under the deposit accession number DSM 33730), or one or more *Lactobacillus plantarum* (in particular, *Lactobacillus plantarum* LB356R, deposited under the accessionaccession number DSM 33094; and/or *Lactobacillus plantarum* LB244R, deposited under the accessionaccession number DSM 32996).

In an embodiment of the present invention the composition comprises, in addition to *Lactobacillus crispatus* LB714R, deposited under the accession number DSM 33732 at least one further probiotic microorganism selected from the group consisting of bacteria, yeasts or molds.

The at least one further probiotic microorganism may be selected from, but not restricted to, *Bifidobacterium lactis* DSM10140, *B. lactis* LKM512, *B. lactis* DSM 20451, *Bifidobacterium bifidum* BB-225, *Bifidobacterium adolescentis* BB-102, *Bifidobacterium breve* BB-308, *Bifidobacterium longum* BB-536 from Zaidanhojin Nihon Bifizusukin Senta (Japan Bifidus Bacteria Center), *Bifidobacterium* NCIMB 41675 described in EP2823822. *Bifidobacterium bifidum* BB-225, *Bifidobacterium adolescentis* BB-102, *Bifidobacterium breve* BB-308, *Bifidobacterium lactis* HN019 (Howaru), *Bifidobacterium bifidum* Bb-02, *Bifidobacterium bifidum* Bb-06, *Bifidobacterium longum* KC-1, *Bifidobacterium longum* 913*, B. lactis* BI-04, *B. lactis* Bi-07 available from DuPont Nutrition Biosciences ApS, *Bifidobacterium lactis* DN 173010 available from Groupe Danone, *Bifidobacterium lactis* Bb-12 available from Chr. Hansen A/S, *Bifidobacterium breve* M-16V (Morinaga) and/or a *Lactobacillus* having a probiotic effect and may be any of the following strains; *Lactobacillus rhamnosus* LGG (Chr. Hansen), *Lactobacillus gasseri* LN40, *L. gasseri* EB01TM, *L. rhamnosus* PB01TM *L. rhamnosus* LN113, *L. fermentum* LN99, *Lactobacillus acidophilus* NCFM, *Lactobacillus bulgaricus* 1260, *Lactobacillus paracasei* Lpc-37, *Lactobacillus rhamnosus* HN001 available from DuPont Nutrition Biosciences ApS, *Streptococcus thermophilus* 715 and *Streptococcus thermophilus* ST21 available from DuPont Nutrition Biosciences ApS, *Lactobacillus paracasei* subsp. *paracasei* CRL431 (ATCC *55544), Lactobacillus paracasei* strain F-19 from Medipharm, Inc. *L. paracasei* LAFTI L26 (DSM Food Specialties, the Netherlands) and *L. paracasei* CRL 431 (Chr. Hansen), *Lactobacillus acidophilus* PTA-4797, *L. salivarius* Ls-33 and *L. curvatus* 853 (DuPont Nutrition Biosciences ApS), *Lactobacillus pentosus* CECT 7504, *Lactobacillus plantarum* 299v (Probi AB), *Lactobacillus plantarum* LMC1 (DSM 32252), *Lactobacillus paracasei* LMC4 (DSM 32254), *Lactobacillus rhamnosus* LMC6 (DSM 32256), *Lactobacillus rhamnosus* LMC7 (DSM 32257), *Lactobacillus paracasei* LMC8 (DSM 32258), *Lactobacillus casei* ssp. *rhamnosus* LC705 is described in Fl Patent 92498, Valio Oy, *Lactobacillus rhamnosus* LC705 (DSM 7061), Propionic acid bacterium e.g. *Propionibacterium freudenreichii ssp.* shermanii PJS (DSM 7067) described in greater details in FI Patent 92498, Valio Oy, Nitrosomonas eutropha D23 (ABIome), *Staphylococcus hominis* strains A9, C2, AMT2, AMT3, AMT4-C2, AMT4-Gl, and/or AMT4-D12. (all from Matrisys Bioscience), *Staphylococcus epidermidis* strains M034, M038, All, AMT1, AMT5-C5, and/or AMT5-G6 (all from Matrisys Bioscience), *L. plantarum* YUN-V2.0 (BCCM LMG P-29456), *L. pentosus* YUN-V1.0 (BCCN LMG P-29455), *L. rhamnosus* YUN-S1.0 (BCCM LMG P-2961) and/or any combinations hereof.

Alternatively, the composition may comprises, in addition to the *Lactobacillus crispatus* according to the present invention, at least one more lactic acid bacteria selected from the group consisting of *Pediococcus pentosaceus* LB606R (DSM 33730), *Lactiplantibacillus plantarum* LB679R (DSM 33731), *Weisella viridescens* LB10G (DSM 32906), *Lactobacillus paracasei* LB113R (DSM 32907), *Lactobacillus paracasei* LB116R (DSM 32908), *Lactobacillus paracasei* LB28R (DSM 32994), *Lactobacillus brevis* LB152G (DSM 32995), *Lactobacillus plantarum* LB244R (DSM 32996), *Lactobacillus plantarum* LB316R (DSM 33091) and *Lactobacillus plantarum* LB356R (DSM 33094).

In a preferred embodiment the composition comprises, in addition to the *Lactobacillus crispatus* according to the present invention, *Pediococcus pentosaceus* (in particular *Pediococcus pentosaceus* LB606R (DSM 33730)), and *Lactobacillus plantarum* (in particular *Lactobacillus plantarum* LB356R (DSM 33094) and/or *Lactobacillus plantarum* LB244R, deposited under the accession number DSM 32996.

A preferred embodiment of the present invention relates to a medical device comprising the composition according to the present invention, or a vaginal microbiome transplant according to the present invention, or an isolated bacterial strain according to the present invention.

The medical device according to the present invention may be formulated into a suspension, spray, gel, cream, lotion, powder, capsule, ointment, oil, solution for lavages, ovules, a vaginal insert, a suppository, lozenge, tablets, microencapsulated product.

Biofilm formation may be prevented by a method for preventing biofilm formation in an environment, wherein the biofilm comprises *Gardnerella* species, such as *Gardnerella vaginalis,* (and/or optionally *candida* species), the method comprising the steps of: administering to the environment an effective amount of a lactic acid bacteria with antimicrobial activity, wherein the environment is a home, workplace, laboratory, industrial environment, aquatic environment, medical device, dental device, epithelial cells, mucous membranes.

The composition according to the present invention, and/or the one or more of the isolated bacterial strains of the present invention may be used in the treatment of one or more of the infections which has arisen from: surgical wounds, decubitus ulcers, infections from catheters, stents, cardiocirculatory devices, prostheses, prosthetic insertions, otologic, orthopaedic and dental prostheses, screws and nails, oral cavity infections and infections of the oral and vaginal mucosa, local infections, otitis, rhinosinusitis, pharyngitis, laryngitis and pneumonia.

In an embodiment of the present invention the composition according to the present invention and/or the one or more of the isolated bacterial strains of the present invention may be effective against antibiotic resistant microorganisms. In particular, effective against an antibiotic resistant infection.

In an embodiment of the present invention the probiotic strain may be used as a live isolated microorganism in a stabilized form. Suitable methods for stabilization are known to those skilled in the art and includes freeze drying or lyophilization involving different cryoprotectants.

In a further embodiment of the present invention the strain may be used as a live isolated strain.

Preferably, the strain may be used as a live isolated stabilized strain. Even more preferably, the strain may be used as a live isolated strain stabilized by lyophilization. Even more preferably, the strain may be used as a live isolated strain stabilized by lyophilization and comprising a cryoprotectant.

### Deposit of biological material

The lactic acid bacteria according to the present invention include in particular microorganisms or analogs, fragments, derivatives, ferments, lysates, mutants or combinations thereof selected from the group comprising the following microorganisms deposited on 14th of December 2020 with the German Collection for Microorganisms and Cell Cultures: *Lactobacillus crispatus* LB714R (DSM 33732).

It should be noted that embodiments and features described in the context of one of the aspects of the present invention also apply to the other aspects of the invention.

The invention will now be described in further details in the following non-limiting examples.

### Examples

### Example 1

### Strain screening identification

### Samples

For identification and selection of microorganisms according to the invention, a strain collection of new isolated lactic acid bacteria (LAB) was established. Samples from different origins, such as homemade sauerkraut, kimchi and healthy human donor samples (vaginal, oral, anal, skin) were collected for isolation of at least 1200 new lactic acid bacteria strains.

A large part of the collection comprises more than 150 strains isolated from women with healthy vaginal microbiota, included is isolates from healthy pregnant women isolated from vagina at late pregnancy.

The samples were collected on Man Rogosa Sharp (MRS, Sigma-Aldrich) broth and agar cultured anaerobically at 37°C overnight or until colony formation. The isolates are plated and sub-cultured until pure colonies were obtained. The pure colonies are stored in MRS broth with 25 % glycerol at -80°C for future use. Strains were identified using 16S rRNA Sanger sequencing standard methods.

### Example 2

### Screening

The +1200 LAB isolated (example 1) was screened for the ability to growth inhibit *Gardnerella vaginitis,* the pathogen being associated with bacterial vaginosis.

*G. vaginalis* JCP7275 Catalog no. HM-1105 was obtained from BEI resources. HM-1105 strain JCP7275, was isolated from a woman tested positive with bacterial vaginosis (Nugent score at 10)
Spot on lawn test for growth inhibition and antimicrobial metabolites were tested using the methods described in Zhang P. et al.(2015) and Arena, M. P. et al.(2016)

The *G*. *vaginalis* was growth on Columbia agar (sigma) for 3-4 days, in an anaerobic camber and at 37°C. Colonies were harvested from the agar plate and inoculated in New York City (NYC) III broth and used directly for spot assay and well diffusion assay.

MIC assay with Cell free supernatant supplemented brain heart infusion glucose medium (sBHIG) was used. It contained 2% (w/v) gelatine, 0.5% yeast extract, 0.1% starch and 1%. HM-1105 was grown in sBHIG overnight directly from the freezer stock.

Bacterial strain isolates from example 1 were cultured from storage samples into 2 mL of MRS broth in 24-well plates.

The well diffusion assay was performed on Columbia agar. An overnight culture of G. *vaginalis* were adjusted to OD 0.1 and 200 µl were used for a lawn. Wells with a diameter at 0.5 cm were punched in the agar. In each well 50 µl of an overnight culture of Lactic acid bacteria were incubated overnight at 37°C under anaerobic conditions. Inhibition zones were measured from the edge of the well to visible growth of *G*. *vaginalis.*

For spot assay *G*. *vaginalis* HM-1105 was cultured in NYCIII broth (ATCC medium: 1685 NYC III) anaerobically for 48-72 hours. The LAB isolates were grown anaerobically in MRS broth overnight at 37°C. The cell density of the overnight culture of *G.vaginalis* is adjusted in NYCIII broth to an optical density at 600 nm (OD600) of 0.1 and hereafter diluted in PBS buffer to a 10⁻² dilution. Two hundred microliters of cell suspension were spread onto Columbia agar plates. The plates with *G*. *vaginalis* lawn were left for drying for approx. 10-20 minutes in sterile air. Three replicates of 20 µL of isolated LAB was spotted onto the G. *vaginalis* lawn. The plates were left for drying and hereafter incubated at 37°C anaerobically for 48-72 hours. The inhibition zone is measured in mm as the clearing zone around the spot, and diameter is measured as the full diameter of the spot. Growth inhibition is observed as the LAB being able to overgrow the *G*. *vaginalis* strain in the spot area indicated in table 2 as a (+). If the *G*. *vaginalis* strain is able to outgrow the spotted bacterial strain, then there is no detected grow inhibition indicated in table 2 as a (-).

2 LAB strains were identified as having a significant growth inhibitory effect on *G*. *vaginalis* and also an inhibition zone of more than 1 mm around spot. Both of these 2 strains were isolated from the vagina of healthy women.

**Table 1**

| Spot assay with type strain *G*. *vaginalis* as target, inhibitor zone measured per strain with an effect. *The diameter was an average of three measurements of the inhibitory zone. **The length of the inhibitor zones was a average of three measurements from the periphery of bacteria spot to growth of *G*. *vaginalis.* | | |
|---|---|---|
| ***G. vaginalis*** | | |
| Strains | *Diameter [mm] | **The inhibitor zone [mm] |
| LB714R | 18 | 3.1 |
| LB919R | 16 | 2.8 |

**Table 2: Growth inhibition and antimicrobial metabolites. Inhibition zone is determined as average of 4 measurements.**

| | Growth inhibition in spot. | | | | Inhibition zone: clearing around the spot (mm) | | | |
|---|---|---|---|---|---|---|---|---|
| LAB | | | | | | | | |
| LB714R | + | + | + | + | 2 | 3 | 2 | 2 |
| LB919R | + | + | + | + | 2 | 2 | 1 | 2 |
| LGG | - | - | - | - | 0 | 0 | 0 | 0 |

### Example 3

### Co-aggregation

*G. vaginalis* HM-1105 was used as research test organism for the screen.

Co-aggregation was determined according to known methods Cisar, J. O. et al. (1979). "Specificity of Coaggregation Reactions between Human Oral Streptococci and Strains of Actinomyces Viscosus or Actinomyces Naeslundii." Infection and Immunity 24 (3): 742-52. Inoculum of all Lactic acid bacteria (LAB) were grown in MRS broth anaerobically overnight and the G. vaginalis was grown on chocolate agar plates anaerobically at 37°C for 48-72 days. The colonies were rinsed, scraped off and suspended in PBS to OD (600 nm) of 0.5 and 1, respectively.

Overnight LAB cell samples were harvest by centrifugation (6000 rpm for 2 min), and supernatants were removed from the pellet. The pellets were washed twice in 1XPBS buffer.

Cell pellet was resuspended in 1XPBS and 500 µl LAB strains were aliquoted into 24 well plates and 500 µl of *G*. *vaginalis* PBS suspension added to each well. The plates were incubated on a shaker (200 rpm). Auto- and co-aggregation formation was observed visually after 1 hour, 2 hours, 3 hours and 24 hours.

As a control for self-aggregation (auto-aggregation), each strain was mixed with PBS in a final volume of 1500 ul in each well.

Co-aggregation was determined by mixing *G*. *vaginalis* and LAB strains. Each strain suspended in PBS buffer, and mixed 1:1 in a final volume of 1500 ul in each well.

The plates were incubated on a shaker at approximately 200 rpm for 24 hours. Co-aggregation formation is observed after 1 hour, 2 hours, 3 hours and 24 hours.

The formation of co-aggregation was scored visually from 1-5 using the following scale:
1: No aggregation
2: Visual initial aggregation
3: Formation of aggregates < 0.5 mm
4: Formation of aggregates > 0.5 mm and < 1 mm
5: Formation of aggregates > 1 mm

5 LAB strains were identified as being able to co-aggregate with *G*. *vaginalis.* The vaginal isolates (LB714R, LB918R, LB919R, LB912R, and LB905R) were found to be superior in the co-aggregation with *G*. *vaginalis.*

LB918R, LB919R, LB912R, and LB905R were isolated from the vagina of a healthy woman. LB905R co-aggregated strongly with *G*. *vaginalis,* with the score of 4 (Fig. 1).

LB714R isolated from vagina of woman in late pregnancy was one of the best strains for co-aggregation with a score of 5 (Fig. 1).

Fig. 1 shows the co-aggregation below the control-row for auto-aggregation. Left well is *G*. *vaginalis* in cell suspension at the concentration of OD 0.5 and right well is with OD 1.

Fig 3 shows co-aggregation scores of LB714R, LB918R, LB919R, LB912R, and LB905R illustrated by the light gray columns determined according to Cisar, J. O. et al. (1979). From the experiments it was demonstrated that LB714R, LB918R, and LB919R showed very strong co-aggregation scores of maximum 5, whereas LB905R was a bit weaker showing a co-aggregation score of 4, and LB912R showed a co-aggregation score of 3.

### Probiotics from commercial products:

Product A: GynoLact available in Denmark by Vitabalans comprising 3 Lactobacillus strains (*L. acidophilis, L. casei, L. rhamnosus*)*.* Pre-culture for test was prepared by breaking one tablet into MRS medium and incubate anaerobically overnight at 37°C.

Product B: Vivag Capsule available in Denmark by Orkla Care A/S comprising 2 Lactobacillus strains (*L. gasseri* EB01TM and *L. rhamnosus* PB01TM). Pre-culture for test was prepared by breaking one tablet into MRS medium and incubate anaerobically overnight at 37°C.

Product C: Ellen available in Denmark by Ellen AB comprising 3 Lactobacillus strains (*L*. *gasseri* LN40, *L. fermentum* LN99, *L. rhamnosus* LN113). Pre-culture for test was prepared by transfer of 0.1 ml to MRS medium and incubate anaerobically overnight at 37°C.

Strains from these 3 commercial products were grown as LAB strains as described above for co-aggregation and all included in the co-aggregation screening as control strains. However, none of these strains have any ability to co-aggregate with *G*. *vaginalis.*

Co-aggregation can inhibit growth of the organism and influence the ability to create biofilm. Thus, LAB being able to co-aggregate with *G*. *vaginalis* can inhibit the biofilm formation and decrease virulence of the strain.

### Example 4

### Competition in co-culture with pathogen

The +1200 LAB isolated (example 1) was screened for the ability to growth inhibit *Gardnerella vaginitis,* the pathogen being associated with bacterial vaginosis.

*G. vaginalis* HM-1105 was obtained from BEI resources.

Competition between LAB and *G*. *vaginalis* was determined according to the methods described in the following publications: Dowarah, R., et al. 2018, Selection and characterization of probiotic lactic acid bacteria and its impact on growth, nutrient digestibility, health and antioxidant status in weaned piglets. PLoS ONE, 13(3), Khare, A., & Tavazoie, S. (2015). Multifactorial Competition and Resistance in a Two-Species Bacterial System. PLoS Genetics, 11(12), 1-21.

*G. vaginalis* was cultured in NYCIII broth (ATCC medium: 1685 NYC III) anaerobically for 48 hours. The cell density of *G*. *vaginalis* and LAB isolates was adjusted according to an optical density at 600 nm (OD₆₀₀) of 1 and harvested by centrifugation (6.000 rpm for two minutes). The cell pellet is washed twice in phosphate buffered saline (1xPBS) and resuspended in 1xPBS. One milliliter of each cell suspensions is mixed in 50 mL of NYCIII broth and co-incubated at 37°C for 24 hours, while monocultures *G*. *vaginalis* and each LAB are used as controls. At time 0, 2 hours, 6 hours, 10 hours and 24 hours, serial dilutions of the cell solutions are plated out on nutrient agar plates to count forming colonies. MRS agar was used for LAB isolates and selective medium Columbia agar base (Merck) with *G*. *vaginalis* selective supplement (Thermo Scientific CM0331 and SR0119) was used for *G*. *vaginalis.*

*Lactobacillus rhamnosus* LGG (Chr. Hansen) was used as a control probiotic strain in all experiments.

20 strains of LAB from the collection of 1200 strains were identified as being able to out-compete growth of *G*. *vaginalis* determined as an ability to decrease growth of the test strain with at least 50%. 6 strains were determined to decrease growth with more than 90%. *L. rhamnosus* LGG as control probiotic was not able to out-compete growth of *G*. *vaginalis* HM-1105.

Only 2 strains, both being isolated from the vagina of healthy women were identified as being superior and able to completely inhibit growth of *G*. *vaginalis.*

The 20 strains identified in example 2 being able to out-compete growth of *G*. *vaginalis* were evaluated for their ability to acidify the substrate in the presence of *G*. *vaginalis.*

Co-culture assay was performed as described in example 2 both with the NYCIII substrate being re-placed by Mueller Hinton broth (Sigma-Aldrich). Initial pH at time of inoculation was 7.4.

After 24 hours of anaerobic incubation growth inhibition in MH broth was determined as described in example 2 and the pH was measured in each MH broth supernatant.

All the 20 strains from example 2 were able to decrease pH to below 4.7.

### Example 5

### Hydrogen peroxide formation

The production of hydrogen peroxide was performed according to the method of Marshall (Marshall, V.M. (1979) J. Appl. Bacteriol. 47 pp 327-328. doi.org/10.1111/j.1365-2672.1979.tb01762.x).

### Example 6

### Prevention of G.vaginalis biofilm

The effect of lactic acid bacteria on prevention of biofilm formation of *G*. *vaginalis* was determined as described in Gottschick et al. (2016) Screening of compounds against Gardnerella vaginalis biofilms. PLos One 11(4). doi.org/10.1371/journal.pone.0154086.

A pre-culture of *G*. *vaginalis* was prepared in NYCIII and incubated 48 hours anaerobic at 37°C. This pre-culture was diluted to OD₆₀₀ = 0.05 in NYCIII (pH 7.0) for the final biofilm culture. The selected lactic acid bacteria were prepared in a similar manner, using MRS for the pre-culture and mixed 1:1 before cultivation in Nunc^{™} MicroWell^{™} 96-Well Microplates (Thermo Scientific). Analysis carried out after 24 hours of incubation at 37°C anaerobically. Supernatant was removed, and biofilms were washed twice with sterile PBS buffer. OD₆₀₀ measured as compared to control with no biofilm and control biofilm of each microorganism grown as a single biofilm culture.

Inhibition of biofilm formation by lactic acid bacteria (LAB) was determined as: 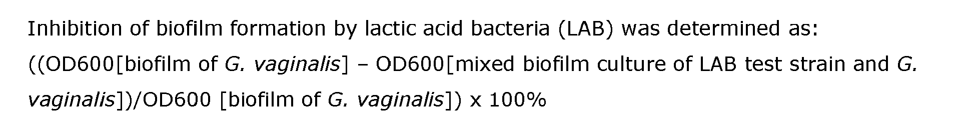 ((OD600[biofilm of G. vaginalis] - OD600[mixed biofilm culture of LAB test strain and G. vaginalis])/OD600 [biofilm of G. vaginalis]) x 100%

The selected strains were compared to commercially available consumer products for vaginal application against bacterial vaginosis.

Product A: GynoLact available in Denmark by Vitabalans comprising 3 Lactobacillus strains (*L. acidophilis, L. casei, L. rhamnosus*)*.* Pre-culture for test was prepared by breaking one tablet into MRS medium and incubate anaerobically overnight at 37°C.

Product B: Vivag Capsule available in Denmark by Orkla Care A/S comprising 2 Lactobacillus strains (*L. gasseri* EB01TM and *L. rhamnosus* PB01TM). Pre-culture for test was prepared by breaking one tablet into MRS medium and incubate anaerobically overnight at 37°C.

Product C: Ellen intimcreme available in Denmark by Ellen AB comprising 3 Lactobacillus strains (*L. gasseri* LN40, *L. fermentum* LN99, *L. rhamnosus* LN113). Pre-culture for test was prepared by transfer of 0.1 ml intimcreme to MRS medium and incubate anaerobically overnight at 37°C.

Each product was prepared for the experiment similar to *G*. *vaginalis* and LAB strains.

| strain | 16S RNA identification (Sanger) | Deposit number | Reduction of biofilm (%) |
|---|---|---|---|
| LB714R | *Lactobacillus crispatus* | DSM 33732 | 95 +/ 7 |
| Product A | mix | Commercial product | 7 +/-12 |
| Product B | mix | Commercial product | 14 +/-25 |
| Product C | mix | Commercial product | 6 +/-19 |

The selected LAB strain has a significant better ability to prevent biofilm formation by G. *vaginalis.*

*Lactobacillus crispatus* LB714R was deposited on December 14^{th} 2020 by Lactobio ApS, Copenhagen, Denmark with the German Collection of Microorganisms and Cell Cultures GmbH (DSMZ) with accession number 33732.

*Lactobacillus gasseri* LB905R, *Lactobacillus crispatus* LB912R, *Lactobacillus jensenii* LB918R and *Lactobacillus crispatus* LB919R were deposited on 25 november 2021 by Lactobio A/S, Copenhagen, Denmark with the German Collection of Microorganisms and Cell Cultures GmbH (DSMZ) as follows: *Lactobacillus gasseri* LB905R deposited under accession number DSM 34094, *Lactobacillus crispatus* LB912R deposited under accession number DSM 34095, *Lactobacillus jensenii* LB918R deposited under accession number DSM 34096, *Lactobacillus crispatus* LB919R deposited under accession number DSM 34097.

### Example 7:

Screenings methods as described in examples 2, 3 and 4 above were repeated using *Candida albicans* as test organism.

### Spot on agar assay:

*Candida* strains were obtained from BEI resources:
*Candida albicans,* strain P57055, catalogue no. 29439 (blood isolate)
*Candida albicans,* strain L26, catalogue no. 29445 (vaginal isolate)
*Candida albicans,* strain 19F, catalogue no. 29449 (vaginal isolate)

Lactic acid bacteria (LAB) strains were incubated in Man Rogosa Sharp (MRS, Sigma-Aldrich) broth overnight at 37 °C. *C. albicans* was incubated overnight at 37 °C in YPD broth (20 g/L peptone, 10 g/L Yeast extract, 20 g/L glucose). The *C*. *albicans* culture with an OD600 of approximately 0.5 was diluted to 10^-1 or 10^-2 and 1 mL was plated on Mueller-Hinton agar plates to create a lawn. From LAB cultures, 20 µL were spotted on top of the Mueller-Hinton plates. Inhibition zones were measured after overnight incubation at 37 °C at aerobic conditions.

For Well diffusion assay, overnight cultures of LAB and *C. albicans* were prepared. LAB was grown in MRS broth and *Candida* was grown in YPD broth at 37 °C. From each of the *Candida* culture with an OD600 of approximately 0.5, a 10^-1 dilution was prepared, and 1 mL was plated on top of Mueller-Hinton agar plates. Wells were made in the plates and 50 µL of the LAB cultures were transferred to the wells. After incubation overnight at 37 °C, inhibition zones around the wells were examined.

Two more strains were identified as active against *Candida albicans.*

The two identified strains were deposited on December 14^{th} 2020 by Lactobio ApS, Copenhagen, Denmark with the German Collection of Microorganisms and Cell Cultures GmbH(DSMZ). Strains were deposited as followed:
*Pediococcus pentosaceus* LB606R deposited as DSM 33730
*Lactiplantibacillus plantarum* LB679R deposited as DSM 33731

*Lactiplantibacillus plantarum* was named according to taxonomy change as released on 15th April 2020 by International Journal of Systematic and Evolutionary Microbiology. Former known as *Lactobacillus plantarum.*

**Table 1: Spot on lawn and diffusion assay, inhibition zones are measured in mm.**

| | Spot on lawn (Diameter in mm) | | Well diffusion (Radius mm) | |
|---|---|---|---|---|
| | LB606R | LB679R | LB606R | LB679R |
| *C. albicans,* strain P57055 | 11 +/- 2 | 14 +/- 3 | 4 +/- 1 | 5 +/- 1 |
| *C. albicans,* strain L26 | 13 +/- 3 | 16 +/- 2 | 2 +/- 1 | 3 +/- 1 |
| *C. albicans,* strain 19F | 8 +/- 2 | 13 +/- 2 | 3 +/- 1 | 3 +/- 1 |

Co-aggregation was for both strain LB606R and LB679R determined to be 3-4.

### Example 8:

Transplantation of vaginal microbiota involving a method for isolation and identification of strains for prevention or treatment of vaginal infections or urogenital infections.

110 healthy women donated swaps taken from their vagina.

The method has been demonstrated in fig. 2 and includes a vaginal transplantation of lactic acid bacteria from a donor with a healthy vaginal microbiota to a patient with a dysfunctional vaginal microbiota.

The method involves a step of isolation, selection and growth of the lactic acid bacteria to be transplanted.

The method involves isolating lactic acid bacteria from the vaginal microbiota of healthy women comprising the following steps:
1. Isolate lactic acid bacteria from vaginal swaps taken from women with healthy vaginal microbiota; swaps are incubated on MRS agar plates, incubated anaerobically at 37°C for 24-48 hours. Colonies are further isolated by striking the colonies on new MRS plates, incubate anaerobically at 37°C for 24-48 hours and each isolated colony is inoculated to 1 ml of MRS broth with 20% glycerol and stored at -80°C.
2. The strain collection from step 1 is screened for antimicrobial activity against either *Gardnerella vaginalis* and/ or *Candida albicans* using the methods described in previously examples. Strains which have at least one antimicrobial activity are selected, the at least one antimicrobial activity is chosen from; growth inhibition, co-aggregation or inhibition of biofilm formation.
3. Strains identified in step 2 are grown as isolated individual strains and in a preferred embodiment the strains are stabilized as individual strains in a composition comprising from 10⁵ to 10¹³ CFU/ml and with a pH from 3 to 7. The composition comprises at least one isolated strain. In one embodiment the composition is a mixture of at least one more isolated strain. The mixture can comprise strains isolated from different donors.
   The composition can be either a solution, oil, wash, gel or ointment for topical application on the epithelia in the vagina or a capsule or tablet for insertion in the vagina. The composition can further comprise prebiotics.
4. The prepared composition of isolated strain(s), isolated from the vagina of healthy women is transferred to a woman with a dysfunctional vaginal microbiota, a vaginal infection, a urinary tract infection, a STIs or to a pregnant woman or a woman in infertility treatment.

### Example 9:

*Lactobacillus crispatus* LB714R, deposited under the accession number DSM 33732, was grown in triplicates, in MRS broth, at 37°C overnight. The grown cells was devide in two fractions, one fraction comprising the viable cells of LB714R, which was evaluated for their ability to co-aggregate with G. vaginalis following same co-aggregation procedure as in example 3, and a second fraction which was centrifuged and the cells removed from the media. The cells were solubilized in PBS buffer with pH 2,2 for 3 days. The cells were spotted on MRS agar to check for their viability to secure that the cells were dead/ inactivated. The dead cells (paraprobiotics) were evaluated for their ability to co-aggregate with *G*. *vaginalis* following same co-aggregation procedure as in example 3.

The viable cells of LB714R showed a strong co-aggregation with a co-aggregation score at 5 and the dead cells of LB714R co-aggregated at an average score of 4.5. (Illustrated in figure 4).

### References

Arena, M. P. et al.(2016) Use of Lactobacillus plantarum Strains as a Bio-Control Strategy against Food-Borne Pathogenic Microorganisms. Frontiers in Microbiology 7 (APR): 1-10. doi.org/10.3389/fmicb.2016.00464.
Dowarah, R., et al. (2018) Selection and characterization of probiotic lactic acid bacteria and its impact on growth, nutrient digestibility, health and antioxidant status in weaned piglets. PLoS ONE, 13(3)
Gottschick et al. (2016) Screening of compounds against Gardnerella vaginalis biofilms. PLos One 11(4). doi.org/10.1371/journal.pone.0154086
Khare, A., & Tavazoie, S. (2015). Multifactorial Competition and Resistance in a Two-Species Bacterial System. PLoS Genetics, 11(12), 1-21.
Kwak, Y-K. Et al. (2016) Persistence of Lactobacilli in postmenopausal women - a double-blind, randomized, pilot study. Gynecol. Obstet Invest 82: 144-150. Doi: 10.1159/000446946
Marshall, V.M. (1979) J. Appl. Bacteriol. 47 pp 327-328. doi.org/10.1111/j.1365-2672.1979.tb01762.x
Zhang P. et al.(2015) Interstrain interactions between bacteria isolated from vacuum-packaged refrigerated beef. Appl Environ Microbiol 81:2753-2761. doi:10.1128/AEM.03933-14

## Claims

1. A composition comprising *Lactobacillus crispatus* LB714R, deposited under the accession number DSM 33732.

2. A vaginal microbiome transplant comprising *Lactobacillus crispatus* LB714R, deposited under the accession number DSM 33732.

3. The vaginal microbiome transplant according to claim 2, wherein the vaginal microbiome transplant further comprises a lactic acid bacterial strain selected from one or more *Pediococcus* strains (such as *Pediococcus pentosaceus* LB606R, deposited under the deposit accession number DSM 33730); and/or the one or more *Lactiplantibacillus* strains, (such as *Lactiplantibacillus plantarum* LB679R, deposited under the deposit accession number DSM 33731).

4. The vaginal microbiome transplant according to anyone of claims 2-3, wherein the vaginal microbiome transplant is formulated as a suspension, spray, gel, cream, lotion, powder, capsule, oil, solution for lavages, ovules, a vaginal insert, a suppository, lozenge, tablets, or microencapsulated product.

5. An isolated bacterial strain selected from *Lactobacillus crispatus,* wherein the isolated bacterial strain is *Lactobacillus crispatus* LB714R, deposited under the accession number DSM 33732.

6. A composition comprising *Lactobacillus crispatus* LB714R, deposited under the accession number DSM 33732 for use as a medicament.

7. A composition comprising *Lactobacillus crispatus* LB714R, deposited under the accession number DSM 33732 for use in the prevention and/or treatment of a vaginal infection in a human or in an animal.

8. A medical device comprising the composition according to anyone of claims 1, or a vaginal microbiome transplant according to claim 2-4, or an isolated bacterial strain according to claim 5.

9. Non therapeutic, in vitro use of *Lactobacillus crispatus* LB714R, deposited under the accession number DSM 33732 for prevention, inhibition or treatment of biofilm formation of pathogenic Gardnerella species.

10. The use according to claim 9, wherein the biofilm formation of pathogenic Gardnerella species is biofilm formation of Gardnerella vaginalis.

11. A food ingredient comprising a composition according to claim 1 or an isolated bacterial strain according to claim 5.

## Patentansprüche

1. Zusammensetzung, umfassend *Lactobacillus crispatus* LB714R, hinterlegt unter der Zugangsnummer DSM 33732.

2. Vaginales Mikrobiomtransplantat, umfassend *Lactobacillus crispatus* LB714R, hinterlegt unter Zugangsnummer DSM 33732.

3. Vaginales Mikrobiomtransplantat nach Anspruch 2, wobei das vaginale Mikrobiomtransplantat ferner einen Milchsäurebakterienstamm umfasst, ausgewählt aus einem oder mehreren *Pediococcus*-Stämmen (wie etwa *Pediococcus pentosaceus* LB606R*,* hinterlegt unter der Hinterlegungszugangsnummer DSM 33730); und/oder dem einen oder den mehreren *Lactiplantibacillus*-Stämmen, (wie etwa *Lactiplantibacillus plantarum* LB679R, hinterlegt unter der Hinterlegungszugangsnummer DSM 33731).

4. Vaginales Mikrobiomtransplantat nach einem der Ansprüche 2-3, wobei das vaginale Mikrobiomtransplantat als eine Suspension, ein Spray, ein Gel, eine Creme, eine Lotion, ein Pulver, eine Kapsel, ein Öl, eine Lösung für Spülungen, Ovula, ein Vaginaleinsatz, ein Suppositorium, eine Lutschtablette, Tabletten oder ein mikroverkapseltes Produkt formuliert ist.

5. Isolierter Bakterienstamm von *Lactobacillus crispatus*, wobei der isolierte Bakterienstamm *Lactobacillus crispatus* LB714R ist, hinterlegt unter der Zugangsnummer DSM 33732.

6. Zusammensetzung, umfassend *Lactobacillus crispatus* LB714R, hinterlegt unter der Zugangsnummer DSM 33732 zur Verwendung als ein Medikament.

7. Zusammensetzung, umfassend *Lactobacillus crispatus* LB714R, hinterlegt unter der Zugangsnummer DSM 33732 zur Verwendung bei der Vorbeugung und/oder der Behandlung einer vaginalen Infektion in einem Menschen oder in einem Tier.

8. Medizinische Vorrichtung, umfassend die Zusammensetzung nach einem der Ansprüche 1, oder ein vaginales Mikrobiomtransplantat nach Anspruch 2-4, oder einen isolierten Bakterienstamm nach Anspruch 5.

9. Nicht therapeutische, in vitro Verwendung von *Lactobacillus crispatus* LB714R, hinterlegt unter der Zugangsnummer DSM 33732 für die Vorbeugung, die Hemmung oder die Behandlung der Biofilmbildung von pathogenen Gardnerella-Spezies.

10. Verwendung nach Anspruch 9, wobei die Biofilmbildung von pathogenen Gardnerella-Spezies die Biofilmbildung von Gardnerella vaginalis ist.

11. Lebensmittelzutat, umfassend eine Zusammensetzung nach Anspruch 1 oder einen isolierten Bakterienstamm nach Anspruch 5.

## Revendications

1. Composition comprenant *Lactobacillus crispatus* LB714R, déposée sous le numéro d'accession DSM 33732.

2. Transplantation de microbiome vaginal comprenant *Lactobacillus crispatus* LB714R, déposée sous le numéro d'accession DSM 33732.

3. Transplantation de microbiome vaginal selon la revendication 2, dans laquelle la transplantation de microbiome vaginal comprend en outre une souche bactérienne d'acide lactique sélectionnée parmi une ou plusieurs souches de *Pediococcus* (telles que *Pediococcus pentosaceus* LB606R*,* déposée sous le numéro d'accession DSM 33730) ; et/ou les une ou plusieurs souches de *Lactiplantibacillus* (telles que *Lactiplantibacillus plantarum* LB679R, déposée sous le numéro d'accession DSM 33731).

4. Transplantation de microbiome vaginal selon l'une quelconque des revendications 2 et 3, dans laquelle la transplantation de microbiome vaginal est formulée sous forme de suspension, de spray, de gel, de crème, de lotion, de poudre, de capsule, d'huile, de solution pour lavages, d'ovules, d'un insert vaginal, d'un suppositoire, d'une pastille, de comprimés ou de produit microencapsulé.

5. Souche bactérienne isolée choisie parmi *Lactobacillus crispatus*, dans laquelle la souche bactérienne isolée est *Lactobacillus crispatus* LB714R, déposée sous le numéro d'accession DSM 33732.

6. Composition comprenant *Lactobacillus crispatus* LB714R, déposée sous le numéro d'accession DSM 33732 pour utilisation en tant que médicament.

7. Composition comprenant *Lactobacillus crispatus* LB714R, déposée sous le numéro d'accession DSM 33732 pour utilisation dans la prévention et/ou le traitement d'une infection vaginale chez un humain ou chez un animal.

8. Dispositif médical comprenant la composition selon l'une quelconque de la revendication 1, ou transplantation de microbiome vaginal selon les revendications 2 à 4, ou souche bactérienne isolée selon la revendication 5.

9. Utilisation non thérapeutique in vitro de *Lactobacillus crispatus* LB714R, déposé sous le numéro d'accession DSM 33732 pour la prévention, l'inhibition ou le traitement de la formation de biofilm d'espèces pathogènes de Gardnerella.

10. Utilisation selon la revendication 9, dans laquelle la formation de biofilm d'espèces pathogènes de Gardnerella est la formation de biofilm de Gardnerella vaginalis.

11. Ingrédient alimentaire comprenant une composition selon la revendication 1 ou souche bactérienne isolée selon la revendication 5.
